# EUROPÄISCHE PATENTSCHRIFT

(11) **EP 0 700 393 B1**
(45) Veröffentlichungstag und Bekanntmachung des Hinweises auf die Patenterteilung: **22.10.1997**
(21) Anmeldenummer: 94915566.7
(22) Anmeldetag: 29.04.1994
(51) Int. Cl.: C07D 257/02, A61K 49/00, A61K 49/04

(54) **FLUORHALTIGE MAKROCYCLISCHE METALLKOMPLEXE**
MACROCYCLIC METAL COMPLEXES CONTAINING FLUORINE
COMPLEXES METALLIQUES MACROCYCLIQUES FLUORES

(30) Priorität: 24.05.1993 DE 4317588
(43) Veröffentlichungstag der Anmeldung: 13.03.1996
(73) Patentinhaber: SCHERING AKTIENGESELLSCHAFT, 13342 Berlin (DE)
(72) Erfinder: PLATZEK, Johannes, D-14195 Berlin (DE); RADÜCHEL, Bernd, D-13465 Berlin (DE); NIEDBALLA, Ulrich, D-14195 Berlin (DE); WEINMANN, Hanns-Joachim, D-14129 Berlin (DE); BAUER, Hans, D-12107 Berlin (DE); ROTH, Klaus, D-14167 Berlin (DE)
(86) Internationale Anmeldenummer: EP9401377
(87) Internationale Veröffentlichungsnummer: WO9427978

(56) Entgegenhaltungen:
- EP-A- 0 255 471
- EP-A- 0 292 689
- EP-A- 0 299 795
- EP-A- 0 434 345
- EP-A- 0 434 346
- EP-A- 0 448 191
- EP-A- 0 485 045
- EP-A- 0 512 661
- WO-A-93/24469

## Beschreibung

Die Erfindung betrifft den in den Patentansprüchen gekennzeichneten Gegenstand, das heißt, fluorhaltige makrocyclische Metallkomplexe, diese Verbindungen enthaltende Mittel, ihre Verwendung als NMR-Diagnostika sowie Verfahren zur Herstellung dieser Verbindungen und Mittel.

Mit Hilfe moderner diagnostischer Methoden gelingt es kleinste morphologische Strukturen mit einer Auflösung darzustellen, die der von Gewebeschnitten aus Anatomielehrbüchern nahekommt. Diese ernorm hohe Auflösung wird zum einen durch eine ständig verbesserte Gerätetechnik, zum anderen aber auch durch den Einsatz von Kontrastmitteln erreicht. Es gelingt jedoch mit den verschiedenen bekannten Methoden wie Ultraschall-, der Röntgen-Diagnostik, der Nuklearmedizin und selbst der Kernspintomographie nicht, Informationen über den stoffwechselphysiologischen Zustand eines Gewebes des lebenden Organismus zu erhalten. Für eine genauere Diagnose und insbesondere für eine Planung und Verlaufskontrolle eines therapeutischen Einsatzes ist jedoch diese Kenntnis von erheblicher Bedeutung, da eine optimale Therapie nur dann erfolgreich sein kann, wenn frühzeitig eine Aussage über deren Wirkung möglich ist.

Es ist bekannt, daß ein wichtiger Faktor der stoffwechselphysiologischen Aktivität die Temperatur ist. Die Bestimmung dieser Gewebe-Temperatur führt zu wichtigen Aussagen über Funktion und Zustand der Zellen, so daß es wünschenswert ist, Orte zu lokalisieren, die eine von der Normalkörpertemperatur abweichende Temperatur haben. Dadurch ist es möglich, pathologisch verändertes Gewebe zu identifizieren und ggf. eine Therapie vorzunehmen.

Die Körpertemperatur ist ein Produkt der Aktivität des Energiestoffwechsels und unterliegt vielfältigen Einflüssen.

Der Blutfluß stellt eine wesentliche Einflußgröße der lokalen Gewebetemperatur dar, durch den der Körper versucht ständig auftretende Temperaturgefälle auszugleichen [K. Brück, Wärmehaushalt und Temperaturregelung, in: Physiologie des Menschen, R.F. Schmidt G. Thews (Hersg.), Springer Verlag, 23. Auflage, 1987]. Die Temperaturmessung bietet daher eine Möglichkeit lokale Mehr- (z.B. bei Entzündungen) oder Minderdurchblutungen (z.B. in ischämischen Gebieten) eines Gewebes gegen seine gesunde Umgebung abzugrenzen.

Bei der Hyperthermiebehandlung von Tumorerkrankungen ist die Messung der Gewebetemperatur eine wichtige Kenngröße zur Verlaufskontrolle der Bestrahlung. Bislang können dafür nur invasive Methoden genutzt werden [P. Fessenden, Direct Temperature Measurement, in: Hyperthermia in Cancer Treatment, Cancer Research, 44 (Suppl.), 4703s-4709s, 1984].

Es ist nun bekannt, daß die chemische Verschiebung von Signalen in der in-vitro NMR-Spektroskopie auch eine Funktion der Temperatur ist. Dieser Einfluß wird durch inter- und intramolekulare Wechselwirkungen hervorgerufen. In der hochauflösenden NMR Spektroskopie bestimmen intermolekulare Wechselwirkungen z.B. mit dem Lösungsmittel ganz entscheidend die chemische Verschiebung. Die Solvatation des untersuchten Moleküls, einschließlich zwischenmolekularer Aggregation und Wasserstoffbrückenbildung, ist stark von der Temperatur abhängig. Wasserstoffbrückenbindungen werden bei höheren Temperaturen aufgebrochen und ändern damit die chemische Umgebung der Atomkerne. Bei Substanzen, die starke intermolekulare Wasserstoffbrückenbindungen ausbilden, ist der Temperaturkoeffizient der chemischen Verschiebung besonders groß. Mit Hilfe von Eichkurven kann dann die Temperatur aus der experimentell gemessenen chemischen Verschiebung genau bestimmt werden. Bewährt haben sich hierbei vor allem die zu starken Wasserstoffbrückenbindungen neigenden aliphatischen Alkohole:

| | |
|---|---|
| Methanol CH₃OH: | T = 409,0 - 36,54 Δδ - 21,85 (Δδ)² |
| Ethylenglykol HOCH₂-CH₂OH: | T = 466,5 - 102,00 Δδ |

wobei Δδ die Differenz der chemischen Verschiebungen zwischen OH- und CH-Signal in ppm und T die absolute Temperatur in K ist [R. Duerst, A. Merbach, Rev. Sci. Instrum. 36, 1896 (1965)].

Die Änderung der chemischen Verschiebung mit der Temperatur durch intermolekulare Wechselwirkung ist keineswegs auf das Proton beschränkt, sondern eine allgemeine Eigenschaft aller magnetisch aktiven Atomkerne, so daß eine ganze Anzahl von Temperaturstandards in der Literatur vorgeschlagen wurden.

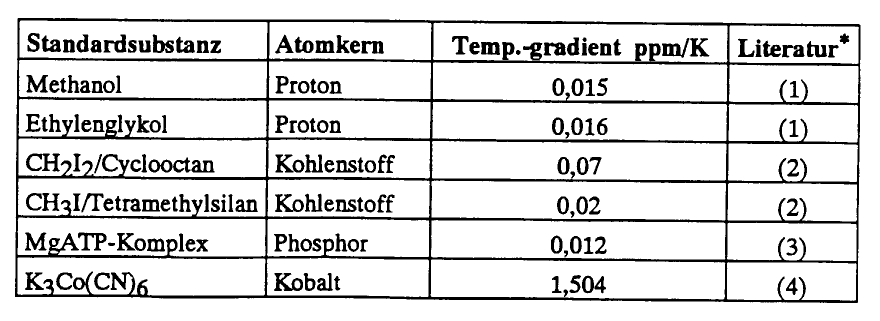

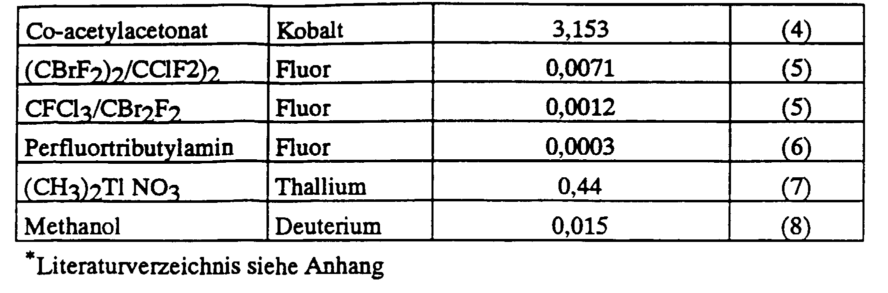

Weiterhin wurde ein ¹³C-NMR-Thermometer vorgeschlagen, das auf der Änderung der Komplexbildungskonstante zwischen dem Verschiebungsreagenz Yb(fod)₃ und Aceton basiert [H.J. Schneider, W. Freitag, M. Schommer, J. Magn. Reson. 18, 393 (1973)]. Dieses Verfahren kann allerdings nur in organischen Lösemitteln angewendet werden.

Der Einfluß von intramolekularen Wechselwirkungen auf die chemische Verschiebung ist bei praktisch allen organischen Verbindungen zu gering, um für Temperaturmessungen herangezogen zu werden. In der Literatur ist nur ein Beispiel dieser Art beschrieben, bei dem die intramolekulare Rotationsbarriere im Furfural und die damit verbundene Linienformveränderungen im ¹³C-NMR-Spekrum zur Temperaturmessung benutzt wurde [S. Combrisson, T. Prange, J. Magn. Reson. 19, 108 (1973)]. Dieses Verfahren eignet sich aber nur in einem sehr schmalen Temperaturbereich und dieser Meßbereich hängt stark von der verwendeten magnetischen Meßfeldstärke ab.

Eine Anwendung dieser Methode zur in-vivo Temperaturmessung von Körpergewebe scheiterte jedoch bislang aus vielfältigen Gründen. So sind die meisten der in der Literatur beschriebenen Verbindungen mit Wasser nicht mischbar, bzw. nur in unpolaren organischen Lösemitteln wie Chloroform löslich. Damit ist ein Einsatz in intakten biologischen Systemen praktisch ausgeschlossen. Zwar gelang die Einbringung einer reinen Perfluortributylaminblase in ein Kaninchenauge und die anschließende Temperaturmessung [B. A. Berkowitz, J.T. Handa, C.A. Wilson, NMR in Biomedicine 5, 65 (1992)], jedoch ist dieses Verfahren hochinvasiv und nicht auf andere Organe übertragbar. Einige der wasserlöslichen Verbindungen wie Methanol, Ethylenglykol, K₃Co(CN)₆ und Thalliumsalze müssen aufgrund der hohen Toxizität von vornherein ausgeschlossen werden. Von den beschriebenen Substanzen ist theoretisch allein der MgATP-Komplex als körpereigene Temperatursonde geeignet. Da die relative MgATP-Konzentration im Cytosol gering ist (um 10 mM/Kg) und wegen des Kreatininkinase-Gleichgewichts durch externe Zugabe nicht erhöht werden kann und weiterhin der Phosphorkem unempfindlich und die chemischen Verschiebungen zusätzlich stark von der Ionenstärke und dem pH des Mediums abhängig sind, ist eine präzise Temperaturbestimmung mit ³¹P-NMR-Messungen am MgATP in vertretbarer Zeit nicht möglich. Somit sind alle in der Literatur beschriebenen Temperatursonden für eine intrazelluläre in vivo Temperaturmessung in der klinischen Routine-Diagnostik ungeeignet.

Aufgabe der vorliegenden Erfindung war es daher geeignete Verbindungen für die in-vivo Temperaturmessung mittels NMR-Spektroskopie zu finden.
Diese Verbindungen müssen die folgenden Anforderungen erfüllen:
a) Sie müssen auf eine Veränderung der Temperatur mit einer veränderten Resonanzfrequenz im NMR-Spektrum reagieren,
b) eine starke chemische Verschiebung pro Grad-Temperaturänderung,
c) eine für diagnostische Anwendungen geeignete Pharmakokinetik,
d) eine für eine Messung genügend hohe Anreicherung in den Zielgeweben,
e) eine gute Verträglichkeit und geringe Toxizität,
f) eine metabolische Stabilität,
g) eine hohe chemische Stabilität und Lagerfähigkeit und
h) gute Wasserlöslichkeit aufweisen.

Die erfindungsgemäßen fluorhaltigen makrocyclischen Metallkomplexe und die aus ihnen bereiteten Lösungen erfüllen die genannten Anforderungen in überraschender Weise. Sie eignen sich darüber hinaus nicht nur hervorragend als Temperatursonden zur Messung der Gewebetemperatur, sondern in gleicher Weise auch als Kontrastmittel bei bildgebenden NMR-Verfahren sowie in der Röntgendiagnostik.

Für die Anwendung der erfindungsgemäßen Komplexverbindungen als Temperatursonde in der NMR-Diagnostik eignen sich bevorzugt Metallkomplexe mit paramagnetischen Metallionen der Elemente der Ordnungszahlen 21-29, 42, 44, 58-70.

Neben den geforderten großen Temperaturgradienten (Punkt b) zeichnen sich die erfindungsgemäßen Verbindungen insbesondere auch durch eine sehr gute Verträglichkeit aus (Punkt e).

Die erfindungsgemäßen fluorhaltigen makrocyclischen Metallkomplexe bestehen aus mindestens einem Metallion eines Elementes der Ordnungszahlen 21-29, 42, 44 oder 57-83 und einem Komplexbildner der allgemeinen Formel I 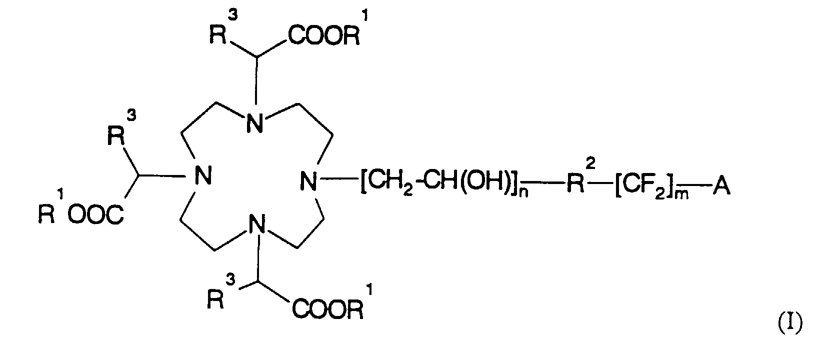 worin
- n und m: unabhängig voneinander für die Ziffern 0 oder 1 steht,
- R¹: unabhängig voneinander für ein Wasserstoffatom oder ein Metallionenäquivalent,
- R³: für ein Wasserstoffatom, eine geradkettige oder verzweigte C₁-C₁₀ Alkylgruppe, die gegebenenfalls durch 1-5 C₁-C₆-Alkoxygruppen, Hydroxy-C₁-C₆-alkylgruppen und/oder Hydroxygruppen substituiert ist,
- R²: für eine durch 1 bis 3 -CF₃ - Gruppen substituierte geradkettige oder verzweigte C₁-C₁₀ Alkylengruppe, die gegebenenfalls durch 1 bis 5 Sauerstoffatome und oder Carbonylgruppen unterbrochen ist und/oder gegebenenfalls durch 1 bis 5 Hydroxygruppen, C₁-C₆-Alkoxy-C₁-C₆-alkylgruppen, -OR⁴ -, -CO-NR⁵R⁶ -, -NR⁵R⁶ - und/oder -NR⁵-CO-R⁶ - Reste substituiert ist,
worin R⁴ für einen geradkettigen oder verzweigten C₁-C₄ Alkylrest steht und R⁵, R⁶ unabhängig voneinander die Bedeutung von R³ haben und
- A: für den Fall, daß m die Ziffer 1 bedeutet, für ein Fluoratom steht, im Falle daß m die Ziffer 0 bedeutet, für ein Wasserstoffatom oder einen zweiten makrocyclischen Rest der allgemeinen Formel II steht, 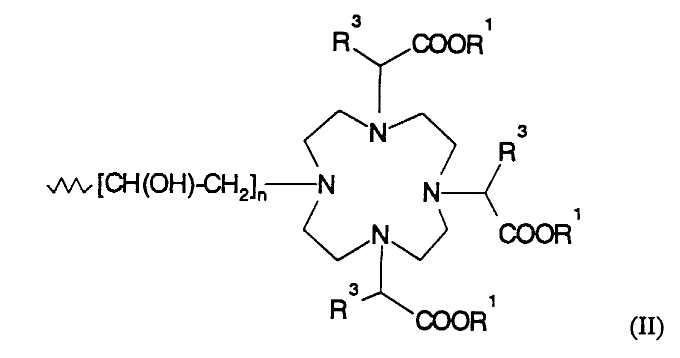
worin
n, R¹ und R³ die angegebenen Bedeutungen haben,
sowie deren Salzen mit anorganischen und/oder organischen Basen oder Aminosäuren und/oder deren C₁-C₆-Alkyl- oder C₆-C₁₀-Aryl- oder Aralky-Estern oder Amiden, wobei mindestens zwei Reste R¹ für ein Metallionenäquivalent stehen.

Bevorzugt sind Verbindungen, bei denen der Rest 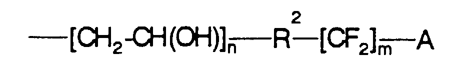 für eine -CH₂-CH(OH)-CF₃ -, -CH₂-C(OH)CF₃-CH₃ -, -CH₂-CH(OH)-C(CF₃)₃ -oder -CH₂-CH(OH)-CH₂O-C(CF₃)₃ - Gruppe steht.

Überraschend ist, daß die chemischen Verschiebungsänderungen allein intramolekularen Ursprungs sind und dadurch unabhängig von externen Einflüssen wie Ionenstärke, pH und Sauerstoffpartialdruck sind, d.h. die Temperaturabhängigkeit der chemischen Verschiebung beruht allein auf der Wechselwirkung zwischen einem Zentralion und den sich im Liganden befindenen Atomkernen.

Überraschend ist weiterhin, daß diese durch intramolekulare Wechselwirkung hervorgerufene chemische Verschiebung ausreichend groß ist, um für eine in-vivo Temperaturmessung herangezogen werden zu können.

Die Temperaturgradienten sind vom gemessenen Atomkern, der chemischen Struktur des Liganden und dem Zentralion abhängig.

Dabei können je nach Komplex und Zentralion positive als auch negative Temperaturgradienten beobachtet werden, daß heißt der Temperatureinfluß auf die chemische Verschiebung der durch die Komplexe hervorgerufenen Signale kann in unterschiedliche Richtungen verlaufen. Damit ergibt sich die Möglichkeit, durch Mischung zweier Komplexe mit unterschiedlichen Vorzeichen in den Temperaturgradienten die Präzision der Temperaturmessung deutlich zu erhöhen.

Ein für die lokalisierte in-vivo NMR-Spektroskopie ganz entscheidender Vorteil dieser Verbindungsklasse liegt darin, daß zur Temperaturbestimmung ausschließlich Signale herangezogen werden, die von den Fluor-Kernen verursacht sind. Da diese Signale in einem völlig anderen spektralen Bereich zu beobachten sind als Signale, die von körpereigenen Substanzen (d.h. im wesentlichen von den Protonen des Gewebewassers) hervorgerufen werden, erlaubt die Verwendung der erfindungsgemäßen Substanzen die nahezu störungsfreie Messung der temperaturempfindlichen Signale.

Ein weiterer Vorteil bietet die große Flexibilität der chemischen Struktur des Liganden, die dem zu lösenden Meßproblem angepaßt werden kann. Durch entsprechende Einstellung der longitudinalen Relaxationszeit T₁ des Meßsignals kann z. B. eine optimale Empfindlichkeit erzielt werden. Für lokalisierte spektroskopische Methoden, die auf einer Spin-Echo-Bildung beruhen, kann auf gleichem Wege die transversale Relaxationszeit T₂ optimal eingestellt werden kann.

In gleicher Weise eignen sich die erfindungsgemäßen Metallkomplexe auch als Kontrastmittel in bildgebenden NMR-Verfahren bzw. in der Röntgendiagnostik, wobei bei den bildgebenden NMR-Verfahren bevorzugt Komplexe mit Metallionen der Elemente der Ordnungszahlen 21-29, 42, 44 oder 57-70, in der Röntgendiagnostik Komplexe mit Metallionen der Elemente der Ordnungszahlen 21-29, 42, 44 oder 57-83 eingesetzt werden.

Die Herstellung der erfindungsgemäßen fluorhaltigen Komplexbildner der allgemeinen Formel I (d.h. von Verbindungen bei denen R¹ für ein Wasserstoffatom steht) erfolgt, indem 1,4,7-Tris(carboxymethyl)-1,4,7,10-tetraazacyclododecan, mit Epoxiden oder Halogenverbindungen alkyliert wird.

Verbindungen der Formel I bei denen R³ ungleich Wasserstoff ist, lassen sich in Analogie zu den in der DE 41 40 779 beschrieben Verfahren herstellen, indem zunächst die Seitenkette -R²-(CF₂)ₘ-A durch Umsetzung des entsprechenden Epoxids mit Tricyclotridecan eingeführt wird. Nach Abspaltung der Formylgruppe des entstanden Intermediates, wird das so erhaltene Produkt mit einer Verbindung der allgemeinen Formel III umgesetzt, 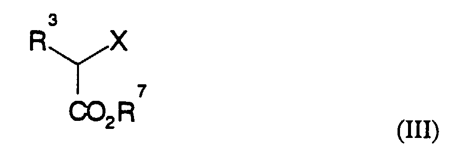 worin X für ein Nucleofug, z.B. für ein Halogen oder einen Sulfonsäurerest und R⁷ für ein Wasserstoffatom oder für eine Säureschutzgruppe, bevorzugt für eine Butylgruppe, steht.

An Stelle des oben eingesetzten Tricyclotridecans kann auch Tris(benzyloxycarbonyl)cyclen mit Epoxiden alkyliert werden (s. J. Chem. Soc. Perkin Trans. I,12, p. 3329 [1991]).

Die für die Umsetzungen benötigten Epoxide sind käuflich (3,3,3-Trifluor-2,3-epoxypropan, Fluorochem Ltd. Derbyshire, U.K.), literaturbekannt (2-Trifluormethyl-2,3-epoxypropan, McBee et al., J. Amer. Chem. Soc. 78, (1956), 4053, 4055, R.H. Groth, J. Org. Chem. 25, (1960), 102, 103.) und leicht erhältlich oder aus käuflichen Vorstufen nach dem Fachmann wohlbekannten Verfahren darstellbar.

So ist der Nonafluor-t-butyl-2,3-epoxypropylether aus dem Perfluor-t-butanol (Fluorochem Ltd. Derbyshire, U.K.) und Epichlorhydrin nach den Verfahren zur Glycidethersynthese (V. Ulbrich u. H. Rejková, Collect. 24 (1959), 2114; Houben-Weyl, Methoden der Organischen Chemie, Bd. VI/3, S. 421 ff., (1965) Georg Thieme Verlag, Stuttgart) erhältlich.

Das 4,4,4-Trifluormethyl-3,3-bis(trifluormethyl)-2,3-epoxypropan wird aus Octafluorcyclobutan (Fluorochem Ltd., Derbyshire, U.K.) über das Perfluorisobutylen (Syntheses of Fluororganic Compounds, Edited by I.L. Knunyants and G.G. Yakoleson, Springer-Verlag Berlin, Heidelberg, New York, Tokyo, p. 9 (1985)) und das 4,4,4-Trifluor-3.3-bis(trifluormethyl)-but-1-en (ibid. p. 11) dargestellt.

Das Olefin wird durch Persäuren wie 3-Chlorperbenzoesäure (Kaufware Fa. Aldrich, Steinheim, Deutschland). F. Fringueli et al., Tetrahedron Letters 30, 1427 (1989), 4-Nitrobenzoesäure (D. Swern et al., Chem. and Ind. 1304, (1962), Trifluorperessigsäure (W. D. Emmons u. A.S. Pogano, Am. Soc. 77, 89, (1955), oder in situ dargestellter Peroxyiminoessigsäure (Y. Ogata und Y. Sawoki, Tetrahedron 20 ,2064, (1964) in das Epoxid überführt.

Für die Umwandlung eines Olefin in ein Epoxid ist auch die Addition von Hypohalogenid zum Halogenhydrin (Houben-Weyl, Methoden der Organischen Chemie, Bd. V/3, S. 762 ff, (1962) Georg Thieme Verlag, Stuttgart), gefolgt von Cyclisierung (Houben-Weyl, Methoden der Organischen Chemie, Bd. VI/3, S. 374 ff, (1965), zum Oxiran geeignet.

Die Umsetzung von 1,4,7-Triscarboxymethyl-1,4,7,10-Tetraazacyclododecan mit Epoxiden erfolgt in Lösungsmitteln bei Temperaturen zwischen 10 und 150°C, bevorzugt bei 50-80°C. Als Lösungsmittel kommen alle inerten Lösungsmittel in Frage. Die Reaktion wird unter Zusatz von Basen ausgeführt. Die Base kann in fester oder gelöster Form zugefügt werden. Als Basen kommen in Betracht Lithiumhydroxid, Natriumhydroxid, Kaliumhydroxid, Alkali- und Erdalkalicarbonate und -oxide, oder organische Basen, wie tertiäre Amine, z.B. Triethylamin oder Diisopropylethylamin, N-Methyl-Morpholin oder Tetramethylpiperidin.

Die Umsetzung von 1,4,7-Triscarboxymethyl-1,4,7,10-tetraazacyclododecan mit Halogenverbindungen kann ebenfalls in fester oder flüssiger Form durchgeführt werden. Bevorzugte Lösungsmittel sind Dioxan, Tetrahydrofuran, Dimethylformamid, Dimethylacetamid und Methanol, Ethanol, Propanol, Isopropanol und Wasser. Die Reaktion erfolgt unter Zusatz von Basen (wie zuvor angegeben) bei Temperaturen zwischen 40°C und 150°C, bevorzugt bei 75° bis 110°C.

Die Herstellung der erfindungsgemäßen Metallkomplexe aus diesen Komplexbildnem erfolgt in der Weise, wie sie in der Deutschen Offenlegungsschrift 34 01 052 und EP 0 450 742 und EP 0 413 405 offenbart worden ist, indem man das Metalloxid oder ein Metallsalz (beispielsweise das Nitrat, Acetat, Carbonat, Chlorid oder Sulfat) des Elements der Ordnungszahlen 21-29, 42, 44, 57-83 in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome durch Kationen anorganischer und/oder organischer Basen oder Aminosäuren substituiert bzw. zu Estem oder Amiden umsetzt.

Die Neutralisation eventuell noch vorhandener freier Säuregruppen erfolgt mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler und saurer Aminosäuren. Altemativ können freie Säuregruppen in an sich bekannter Weise ganz oder teilweise in Ester- oder Amidgruppen überführt werden.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufallen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es of zweckmäßig, neutrale Mischsalze herzustellen, die sowohl anorganische als auch organische Kationen als Gegenionen enthalten.

Die erfindungsgemäßen pharmazeutischen Mittel werden vorzugsweise in einer Konzentration von 1 µmol-1 mol/l hergestellt. Sie werden in der Regel in Mengen von 0,005-20 mmol/kg Körpergewicht, vorzugsweise 0,05-5 mmol/kg Körpergewicht, dosiert. Sie sind zur enteralen und parenteralen Applikation bestimmt. Die erfindungsgemäßen Mittel erfüllen die vielfältigen Voraussetzungen für die Eignung als Diagnostika für die NMR-Tomographie und -Spektroskopie. Ferner zeigen sie die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit der Komplexe und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen. Damit sind die erfindungsgemäßen Komplexe und Mittel nicht nur als Temperatursonden in der NMR-Spektoskopie hervorragend geeignet, sondern auch als Kontrastmittel bei bildgebenden NMR-Methoden sowie in der Röntgendiagnostik.

Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen gebundenen Ionen innerhalb der Zeit, in der die neuen Kontrastmittel vollständig wieder ausgeschieden werden, nur äußerst langsam erfolgt.

Durch die Verwendung der sehr gut verträglichen Komplexe als neuartige Meßsonden ist es möglich geworden, in kleineren Volumina (z. B. 10 cm³) ortsaufgelöste Spektroskopie durchzuführen und die Temperatur präzise in kurzer Meßzeit ohne Störung bzw. Überlagerung durch andere Moleküle zu bestimmen. Für ein in-vivo-Imaging (NMR-Bildgebung) sind die genannten Verbindungen ebenfalls geeignet.

Die nachfolgenden Beispiele dienen der Erläuterung des Erfindungsgegenstandes, ohne ihn auf diese beschränken zu wollen.

### Beispiel 1

a) 10-(2-Hydroxy-2-trifluormethyl-ethyl)-1,4,7-tris-carboxymethyl-1,4,7,10-tetraazacyclododecan
12,94 g (115,44 mmol) (1,1,1-Trifluor-2,3-epoxypropan) und 10 g (28,86 mmol) 1,4,7-Triscarboxymethyl-1,4,7,10-tetraazacyclododecan werden in einer Mischung aus 50 ml Dioxan/80 ml Wasser gelöst, und der pH-Wert mit 6 N Kalilauge auf pH 10 gebracht. Man rührt 24 Stunden bei 70°C unter Druckabschluß. Man dampft zur Trockne ein, nimmt den Rückstand mit 300 ml Wasser/50 ml Methanol auf und extrahiert 2 mal mit 100 ml tert.-Butyl-methylether. Die wäßrige Lösung wird mit 5 N-Salzsäure auf pH 1 gestellt und zur Trockne eingedampft. Der Rückstand wird mit 200 ml Methanol/80 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser/20 ml Ethanol gelöst und anschließend auf eine Säule aus Poly-(4-vinylpyridin) gegeben. Das Produkt wird mit einer Lösung aus Ethanol/Wasser 1:3 eluiert. Nach Eindampfen im Vakuum wird der Rückstand an einer Reversed Phase-Säule (RP 18/Laufmittel= Gradient aus Wasser/Tetrahydrofuran) chromatographiert. Nach Eindampfen der Hauptfraktion erhält man 10,81 g (68 % d. Th.) eines stark hygroskopischen, glasigen Feststoffes.
Wassergehalt: 11,3 %

| Analyse (bezogen auf wasserfreie Substanz): | | | | |
|---|---|---|---|---|
| ber. | C 44,54 | H 6,38 | N 12,22 | F 12,43 |
| gef. | C 44,31 | H 6,51 | N 12,12 | F 12,19 |

b) Lanthan-Komplex von 10-(2-Hydroxy-2-trifluormethyl-ethyl)-1,4,7-tris-carboxymethyl-1,4,7,10-tetraazacyclododecan
4,69 g (10,24 mmol) der Titelverbindung aus Beispiel la werden in 50 ml Wasser gelöst und 1,67 g (5,12 mmol) Lanthanoxid zugegeben. Man rührt 3 Stunden bei 90°C. Die Lösung wird eine Stunde mit 2 ml saurem Ionenaustauscher (ANB 252c/H⁺-Form) und 2 ml schwach basischem Austauscher IRA 67/OH⁻-Form) bei Raumtemperatur gerührt. Es wird vom Austauscher abfiltriert und das Filtrat gefriergetrocknet.
Ausbeute: 6,53 g (94 % d. Th.) eines glasigen Feststoffes
Wassergehalt: 8,0 %

| Analyse (bezogen auf wasserfreie Substanz): | | | | | |
|---|---|---|---|---|---|
| ber. | C 34,36 | H 4,41 | N 9,43 | F 9,59 | La 23,37 |
| gef. | C 34,21 | H 4,50 | N 9,33 | F 9,40 | La 22,09 |

c) Praseodym-Komplex von 10-(2-Hydroxy-2-trifluormethyl-ethyl)-1,4,7-tris-carboxymethyl-1,4,7,10-tetraazacyclododecan
5,28 g (11,51 mmol) der Titelverbindung aus Beispiel la werden in 50 ml Wasser gelöst und 3,66 g (11,51 mmol) Praseodym(III)acetat zugesetzt. Man erwärmt 2 Stunden auf 90°C und dampft anschließend im Vakuum zur Trockne ein. Der Rückstand wird in 50 ml Wasser aufgenommen und erneut zur Trockne eingedampft. Der Rückstand wird in 100 ml Wasser gelöst und 1 Stunde mit 2 ml saurem Ionenaustauscher (AMB 252c/H⁺-Form) und 5 ml schwach basischem Austauscher (IRA 67/OH⁻-Form) gerührt. Es wird vom Austauscher abfiltriert und das Filtrat gefriergetrocknet.
Ausbeute: 95 % d. Th. eines leicht grünlichen Pulvers

| Analyse: | | | | | |
|---|---|---|---|---|---|
| ber. | C 34,24 | H 4,39 | N 9,40 | F 9,56 | Pr 23,63 |
| gef. | C 34,51 | H 4,20 | N 9,62 | F 9,80 | Pr 23,40 |

d) Dysprosium-Komplex von 10-(2-Hydroxy-2-trifluormethyl-ethyl)-1,4,7-tris-carboxymethyl-1,4,7,10-tetraazacyclododecan
Analog zu 1b wurde Dysprosiumoxid anstelle von Lathanoxid eingesetzt.
Ausbeute: 95 % d. Th. eines farblosen amorphen Pulvers
Wassergehalt: 7,5 %

| Analyse (bezogen auf wasserfreie Substanz): | | | | | |
|---|---|---|---|---|---|
| ber. | C 33,05 | H 4,24 | N 9,07 | F 9,23 | Dy 26,23 |
| gef. | C 33,15 | H 4,36 | N 8,80 | F 9,01 | Dy 26,10 |

e) Europium-Komplex von 10(2-Hydroxy-2-trifluormethyl-ethyl)-1,4,7-tris-carboxymethyl-1,4,7,10-tetraazacyclododecan
Analog zu Beispiel 1b wurde Europiumoxid anstelle von Lanthanoxid eingesetzt.
Ausbeute: 94 % d. Th. eines farblosen Pulvers
Wassergehalt: 7,1 %

| Analyse (bezogen auf wasserfreie Substanz): | | | | | |
|---|---|---|---|---|---|
| ber. | C 33,62 | H 4,31 | N 9,22 | F 9,38 | Eu 25,02 |
| gef. | C 33,30 | H 4,58 | N 9,33 | F 9,09 | Eu 24,88 |

### Beispiel 2

a) 10-(2-Hydroxy-2-trifluormethyl-propyl)-1,4,7-tris-carboxymethyl-1,4,7,10-tetraazacyclododecan
10,29 g (86,58 mmol) 2,3-Epoxy-2-trifluormethylpropan und 10 g (28,86 mmol) 1,4,7-Triscarboxymethyl-1,4,7,10-tetraazacyclododecan werden in einer Mischung aus 50 ml Dioxan/80 ml Wasser gelöst, und der pH-Wert mit 6 N Kalilauge auf pH 10 gebracht. Man rührt 24 Stunden bei 70°C (unter Druckabschluß). Man dampft zur Trockne ein, nimmt den Rückstand mit 300 ml Wasser/50 ml Methanol auf und extrahiert 2 mal mit 100 ml tert.-Butyl-methylether. Die wäßrige Lösung wird mit 5 N-Salzsäure auf pH 1 gestellt und zur Trockne eingedampft. Der Rückstand wird mit 200 ml Methanol/80 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser/20 ml Ethanol gelöst und anschließend auf eine Säule aus Poly-(4-vinylpyridin) gegeben. Das Produkt wird mit einer Lösung aus Ethanol/Wasser 1:3 eluiert. Nach Eindampfen im Vakuum wird der Rückstand an einer Reversed Phase-Säule (RP 18/Laufmittel= Gradient aus Wasser/Tetrahydrofuran) chromatographiert. Nach Eindampfen der Hauptfraktion erhält man 11,04 g (71 % d. Th.) eines stark hygroskopischen, glasigen Feststoffes.
Wassergehalt: 9,3 %

| Analyse (bezogen auf wasserfreie Substanz): | | | | |
|---|---|---|---|---|
| ber. | C 45,76 | H 6,01 | N 18,86 | F 12,06 |
| gef. | C 45,81 | H 6,29 | N 18,99 | F 11,87 |

b) Dysprosium-Komplex von 10-(2-Hydroxy-2-trifluormethyl-propyl)-1,4,7-triscarboxymethyl-1,4,7,10-tetraazacyclododecan
5 g (10,24 mmol) der Titelverbindung aus Beispiel 2a werden in 50 ml Wasser gelöst und 1,91 g (5,12 mmol) Samariumoxid zugegeben. Man rührt 3 Stunden bei 90°C. Die Lösung wird eine Stunde mit 2 ml saurem Ionenaustauscher (ANB 252c/H⁺-Form) und 2 ml schwach basischem Austauscher IRA 67/OH⁻-Form) bei Raumtemperatur gerührt. Es wird vom Austauscher abfiltriert und das Filtrat gefriergetrocknet.
Ausbeute: 6,70 g (96 % d. Th.) eines glasigen Feststoffes
Wassergehalt: 7,3 %

| Analyse (bezogen auf wasserfreie Substanz): | | | | | |
|---|---|---|---|---|---|
| ber. | C 34,21 | H 4,47 | N 8,87 | F 9,02 | Dy 25,71 |
| gef. | C 34,10 | H 4,59 | N 8,63 | F 8,82 | Dy 25,50 |

c) Europium-Komplex von 10-(2-Hydroxy-2-trifluormethyl-propyl)-1,4,7,-tris(carboxymethyl)-1,4,7,10-tetraazacyclododecan
Analog zu Beispiel 2b wurde Europiumoxid anstelle von Dysprosiumoxid eingesetzt.
Ausbeute: 96 % d. Th. eines farblosen amorphen Pulvers
Wassergehalt 8,1 %

| Analyse (bezogen auf wasserfreie Substanz): | | | | | |
|---|---|---|---|---|---|
| ber. | C 34,79 | H 4,54 | N 9,02 | F 9,17 | Eu 24,45 |
| gef. | C 34,59 | H 4,67 | N 8,87 | F 9,05 | Eu 24,23 |

d) Praseodym-Komplex von 10-(2-Hydroxy-2-trifluormethyl-propyl)-1,4,7,-tris(carboxymethyl)-1,4,7,10-tetraazacyclododecan
5,44 g (11,51 mmol) der Titelverbindung aus Beispiel 2a werden in 50 ml Wasser gelöst und 3,66 g (11,51 mmol) Praseodym(III)acetat zugesetzt. Man erwärmt 2 Stunden auf 90°C und dampft anschließend im Vakuum zur Trockne ein. Der Rückstand wird in 50 ml Wasser aufgenommen und erneut zur Trockne eingedampft. Der Rückstand wird in 100 ml Wasser gelöst und 1 Stunde mit 2 ml saurem Ionenaustauscher (AMB 252c/H⁺-Form) und 5 ml schwach basischem Austauscher (IRA 67/OH⁻-Form) gerührt. Es wird vom Austauscher abfiltriert und das Filtrat gefriergetrocknet.
Ausbeute: 92 % d. Th. eines grünlich gefärbten Pulvers
Wassergehalt: 7,3 %

| Analyse (bezogen auf wasserfreie Substanz): | | | | | |
|---|---|---|---|---|---|
| ber. | C 35,42 | H 4,62 | N 9,18 | F 9,34 | Pr 23,09 |
| gef. | C 35,21 | H 4,78 | N 9,03 | F 9,15 | Pr 22,88 |

### Beispiel 3

a) t-(Nonafluor)-butyl-2,3-epoxypropylether
In 100 ml absolutem Ether werden 24,78 g (105 mmol) Nonafluor-t-butanol gelöst und anteilweise mit 2,30 g (100 mmol) Natrium versetzt. Nachdem alles Natrium gelöst ist, versetzt man anteilweise mit 9,25 g (100 mmol) Epichlorhydrin und rührt zunächst 1 Stunde bei Raumtemperatur, danach 2 Stunden bei Rückflußtemperatur. Man destilliert den Ether über eine Kolonne ab und erwärmt den Rückstand 1 Stunde auf 60°C. Nach dem Abkühlen saugt man vom Feststoff ab und destilliert das Filtrat bei leichtem Vakuum (30 m bar, 140°C Bad) im Kugelrohr.
Ausbeute: 23,35 g (21,4 % d. Th.) Gehalt nach GC: 98,2 %

| Analyse (bezogen auf wasserfreie Substanz): | | | |
|---|---|---|---|
| ber. | C 28,78 | H 1,73 | F 58,54 |
| gef. | C 28,62 | H 1,78 | F 58,63 |

b) 10-(2-Hydroxy-3-(tert.-nonafluor-butoxy-propyl)-1,4,7-tris-carboxymethyl-1,4,7,10-tetraazacyclododecan
25,29 g (86,58 mmol) der Titelverbindung aus Beispiel 3a und 10 g (28,86 mmol) 1,4,7-Triscarboxymethyl-1,4,7,10-tetraazacyclododecan werden in einer Mischung aus 50 ml Dioxan/80 ml Wasser gelöst, und der pH-Wert mit 6 N Kalilauge auf pH 10 gebracht. Man rührt 24 Stunden bei 70°C (unter Druckabschluß). Man dampft zur Trockne ein, nimmt den Rückstand mit 300 ml Wasser/50 ml Methanol auf und extrahiert 2 mal mit 100 ml tert.-Butyl-methylether. Die wäßrige Lösung wird mit 5 N-Salzsäure auf pH 1 gestellt und zur Trockne eingedampft. Der Rückstand wird mit 200 ml Methanol/80 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser/20 ml Ethanol gelöst und anschließend auf eine Säule aus Poly-(4-vinylpyridin) gegeben. Das Produkt wird mit einer Lösung aus Ethanol/Wasser 1:3 eluiert. Nach Eindampfen im Vakuum wird der Rückstand an einer Reversed Phase-Säule (RP 18/Laufmittel= Gradient aus Wasser/Tetrahydrofuran) chromatographiert. Nach Eindampfen der Hauptfraktion erhält man 10,55 g (63 % d. Th.) eines stark hygroskopischen, glasigen Feststoffes.
Wassergehalt: 9,1%

| Analyse (bezogen auf wasserfreie Substanz): | | | | |
|---|---|---|---|---|
| ber. | C 39,50 | H 4,89 | N 8,77 | F 26,78 |
| gef. | C 39,31 | H 4,99 | N 8,51 | F 26,57 |

c) Dysprosiumkomplex von 10-(2-Hydroxy-3-(tert.-nonafluor-butoxy)-propyl)-1,4,7,-tris(carboxymethyl)-1,4,7,10-tetraazacyclododecan
5 g (7,83 mmol) der Titelverbindung aus Beispiel 3b werden in 50 ml Wasser gelöst und 1,46 g (3,91 mmol) Dysprosiumoxid zugegeben. Man rührt 3 Stunden bei 90°C. Die Lösung wird eine Stunde mit 2 ml saurem Ionenaustauscher (AMB 252c/H⁺-Form) und 2 ml schwach basischem Austauscher (IRA 67/OH⁻-Form) bei Raumtemperatur gerührt. Es wird vom Austauscher abfiltriert und das Filtrat gefriergetrocknet.
Ausbeute: 6,25 g (93 % d. Th.) eines glasigen Feststoffes
Wassergehalt: 7,0 %

| Analyse (bezogen auf wasserfreie Substanz): | | | | | |
|---|---|---|---|---|---|
| ber. | C 31,61 | H 3,54 | N 7,02 | F 21,43 | Dy 20,36 |
| gef. | C 31,42 | H 3,71 | N 6,90 | F 21,20 | Dy 20,18 |

d) Europium-Komplex von 10-(2-Hydroxy-3-(tert.-nonafluor-butoxy)-propyl)-1,4,7,-tris-(carboxymethyl)-1,4,7,10-tetraazacyclododecan
Analog zu Beispiel 3c wurde Europiumoxid anstelle von Dysprosiumoxid eingesetzt.
Ausbeute: 97 % d. Th. eines farblosen amorphen Pulvers
Wassergehalt: 6,9 %

| Analyse (bezogen auf wasserfreie Substanz): | | | | | |
|---|---|---|---|---|---|
| ber. | C 32,03 | H 3,58 | N 7,12 | F 21,71 | Eu 19,30 |
| gef. | C 31,88 | H 3,70 | N 7,01 | F 21,50 | Eu 19,09 |

e) Praseodym-Komplex von 10-(2-Hydroxy-3-(tert.-nonafluor-butoxy)propyl)-1,4,7,-tris-(carboxymethyl)-1,4,7,10-tetraazacyclododecan
7,35 g (11,51 mmol) der Titelverbindung aus Beispiel 3b werden in 50 ml Wasser gelöst und 3,66 g (11,51 mmol) Praseodym(III)acetat zugesetzt. Man erwärmt 2 Stunden auf 90°C und dampft anschließend im Vakuum zur Trockne ein. Der Rückstand wird in 50 ml Wasser aufgenommen und erneut zur Trockne eingedampft. Der Rückstand wird in 100 ml Wasser gelöst und 1 Stunde mit 2 ml saurem Ionenaustauscher (AMB 252c/H⁺-Form) und 5 ml schwach basischem Austauscher (IRA 67/OH⁻.Form) gerührt. Es wird vom Austauscher abfiltriert und das Filtrat gefriergetrocknet.
Ausbeute: 94 % d. Th. eines grünen Feststoffes
Wassergehalt: 3,5 %

| Analyse (bezogen auf wasserfreie Substanz): | | | | | |
|---|---|---|---|---|---|
| ber. | C 32,49 | H 3,64 | N 7,22 | F 22,02 | Pr 18,15 |
| gef. | C 32,28 | H 3,78 | N 7,05 | F 21,88 | Pr 17,92 |

### Beispiel 4

a) 4,4,4-Trifluor-3,3-bis(trifluormethyl)-1,2-epoxybutan
In einer Lösung von 24,6 g (100 mmol) 4,4,4-Trifluor-3,3-bis(trifluormethyl)-but-1-en in 150 ml absolutem Diethylether werden unter Rühren und Kühlung mit Wasser anteilweise 32,9 g (105 mmol) 3-Chlorperbenzoesäure mit einem Persäuregehalt von 55 % gegeben. Nach beendeter Zugabe wird 3 Stunden am Rückfluß erhitzt. Man läßt erkalten und rührt die Lösung in kalte, gesättigte Natriumhydrogenkarbonatlösung ein. Die etherische Lösung wird abgetrennt, mit Natriumchloridlösung gewaschen, über Natriumsulfat getrocknet und über eine Kolonne eingeengt. Der Rückstand wird durch Kugelrohrdestillation (Vakuum 30 m bar, 130°C Bad) gereinigt.
Ausbeute: 18,95 g (73,3 % d. Th.) Gehalt nach GC: 98,7 %

| Analyse (bezogen auf wasserfreie Substanz): | | | |
|---|---|---|---|
| ber. | C 27,50 | H 1,15 | F 65,24 |
| gef. | C 27,63 | H 1,22 | F 65,12 |

b) 10-(2-Hydroxy-3,3,3-tris(trifluormethyl)-propyl)-1,4,7-tris-carboxymethyl-1,4,7,10-tetraazacyclododecan
22,69 g (86,58 mmol) der Titelverbindung aus Beispiel 4a und 10 g (28,86 mmol) 1,4,7-Triscarboxymethyl-1,4,7,10-tetraazacyclododecan werden in einer Mischung aus 50 ml Dioxan/80 ml Wasser gelöst, und der pH-Wert mit 6 N Kalilauge auf pH 10 gebracht. Man rührt 24 Stunden bei 70°C unter Druckabschluß. Man dampft zur Trockne ein, nimmt den Rückstand mit 300 ml Wasser/50 ml Methanol auf und extrahiert 2 mal mit 100 ml tert.-Butyl-methylether. Die wäßrige Lösung wird mit 5 N-Salzsäure auf pH 1 gestellt und zur Trockne eingedampft. Der Rückstand wird mit 200 ml Methanol/80 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser/20 ml Ethanol gelöst und anschließend auf eine Säule aus Poly-(4-vinylpyridin) gegeben. Das Produkt wird mit einer Lösung aus Ethanol/Wasser 1:3 eluiert. Nach Eindampfen im Vakuum wird der Rückstand an einer Reversed Phase-Säule (RP 18/laufmittel= Gradient aus Wasser/Tetrahydrofuran) chromatographiert. Nach Eindampfen der Hauptfraktion erhält man 13,87 g (71 % d. Th.) eines stark hygroskopischen, glasigen Feststoffes.
Wassergehalt: 10,1%

| Analyse (bezogen auf wasserfreie Substanz): | | | | |
|---|---|---|---|---|
| ber. | C 39,48 | H 4,80 | N 9,21 | F 28,10 |
| gef. | C 39,29 | H 4,95 | N 9,05 | F 28,03 |

c) Dysprosium-Komplex von 10-(2-Hydroxy-3,3,3-tris(trifluormethyl)-propyl)-1,4,7-tris(carboxymethyl)-1,4,7,10-tetraazacyclododecan
5 g (8,22 mmol) der Titelverbindung aus Beispiel 4b werden in 50 ml Wasser gelöst und 1,53 g (4,11 mmol) Dysprosiumoxid zugegeben. Man rührt 3 Stunden bei 90°C. Die Lösung wird eine Stunde mit 2 ml saurem Ionenaustauscher (AMB 252c/H⁺-Form) und 2 ml schwach basischem Austauscher (IRA 67/OH⁻-Form) bei Raumtemperatur gerührt. Es wird vom Austauscher abfiltriert und das Filtrat gefriergetrocknet.
Ausbeute: 6,43 g (95 % d. Th.) eines glasigen Feststoffes
Wassergehalt: 6,7,%

| Analyse (bezogen auf wasserfreie Substanz): | | | | | |
|---|---|---|---|---|---|
| ber. | C 31,28 | H 3,41 | N 7,30 | F 22,27 | Dy 21,16 |
| gef. | C 31,04 | H 3,51 | N 7,14 | F 22,11 | Dy 21,04 |

d) Europium-Komplex von 10-(2-Hydroxy-3,3,3-tris(trifluormethyl)-propyl)-1,4,7-tris(carboxymethyl)-1,4,7,10-tetraazacyclododecan
Analog zu Beispiel 4c wurde Europiumoxid anstelle von Dysprosiumoxid eingesetzt.
Ausbeute: 97 % d. Th. eines farblosen amorphen Pulvers
Wassergehalt: 7,2 %

| Analyse (bezogen auf wasserfreie Substanz): | | | | | |
|---|---|---|---|---|---|
| ber. | C 31,72 | H 3,46 | N 7,40 | F 22,58 | Eu 20,06 |
| gef. | C 31,51 | H 3,59 | N 7,21 | F 22,35 | Eu 19,87 |

e) Praseodym-Komplex von 10-(2-Hydroxy-3,3,3-tris(trifluommethyl)-propyl)-1,4,7-tris(carboxymethyl)-1,4,7,10-tetraazacyclododecan
7,00 g (11,51 mmol) der Titelverbindung aus Beispiel 4b werden in 50 ml Wasser gelöst und 3,66 g (11,51 mmol) Praseodym(III)acetat zugesetzt. Man erwärmt 2 Stunden auf 90°C und dampft anschließend im Vakuum zur Trockne ein. Der Rückstand wird in 50 ml Wasser aufgenommen und erneut zur Trockne eingedampft. Der Rückstand wird in 100 ml Wasser gelöst und 1 Stunde mit 2 ml saurem Ionenaustauscher (AMB 252c/H⁺-Form) und 5 ml schwach basischem Austauscher (IRA 67/OH⁻.Form) gerührt. Es wird vom Austauscher abfiltriert und das Filtrat gefriergetrocknet.
Ausbeute: 94 % d. Th. eines hellgrünen Pulvers
Wassergehalt: 9,5 %

| Analyse (bezogen auf wasserfreie Substanz): | | | | | |
|---|---|---|---|---|---|
| ber. | C 32,19 | H 3,51 | N 7,51 | F 22,91 | Pr 18,88 |
| gef. | C 32,03 | H 3,63 | N 7,38 | F 22,70 | Pr 18,64 |

### Beispiel 5

Für den Praseodym- bzw. für den Dysprosium-Komplex des 10-(2-Hydroxy-2-trifluormethyl-ethyl)1,4,7-triscarboxymethyl-1,4,7,10-tetraazacyclododecans (hergestellt nach Beispiel 1c bzw. 1d) wurden die in der Tabelle 1 zusammengestellten Parameter bestimmt.

**Tabelle 1**

| Beispiel Nr. | 1c | 1d |
|---|---|---|
| Metallatom | Praseodym | Dysprosium |
| Temp. Gradient [ppm/K] | 0,016 | 0,49 |
| chem. Verschiebung δ[ppm] | -82,6 | -160 |
| Linienbreite Δν_{1/2}[Hz] | 35 | 300 |
| rel. Genauigkeit | 0,45 · 10⁻³ | 1,4·10⁻³ |

Die Messungen wurden an einem Bruker Gerät AC 250 im Temperaturbereich zwischen 26°C und 46°C an einer 0,01 molaren wässrigen Lösung des jeweiligen Komplexes durchgeführt. Die chemischen Verschiebungen beziehen sich auf CFCl₃ als externer Standard.

### Literaturverzeichnis:

(1) R. Duerst et al., Rev. Sci. Instrum. **36** (1965) 1896,
   F. Conti, Rev. Sci. Instrum. **38** (1967) 128,
   A.L. Van Geet, Anal. Chem. **40** (1968) 2227,
   A.L. Van Geet, Anal. Chem. **42** (1970) 678,
   C. Ammann et al., J. Magn. Reson. **46** (1982) 319;
(2) D.R. Vidrin et al., Anal. Chem. **48**(1976) 1301;
(3) R.K Gupta et al., J. Magn. Reson. **40** (1980) 587;
(4) J.T. Bailey et al., J. Magn. Reson. **37** (1980) 353;
(5) P.E. Peterson, Anal. Chem. **50** (1978) 298;
(6) B.A. Berkowitz et al., NMR in Biomedicine **5** (1992) 65;
(7) M.J. Foster et al., J. Magn. Reson. **65** (1985) 497;
(8) K. Roth, Magn. Reson. Chem. **25**(1987) 429.

## Patentansprüche

1. Fluorhaltige makrocyclische Metallkomplexe bestehend aus mindestens einem Metallion eines Elementes der Ordnungszahlen 21-29, 42, 44 oder 57-83 und einem Komplexbildner der allgemeinen Formel I 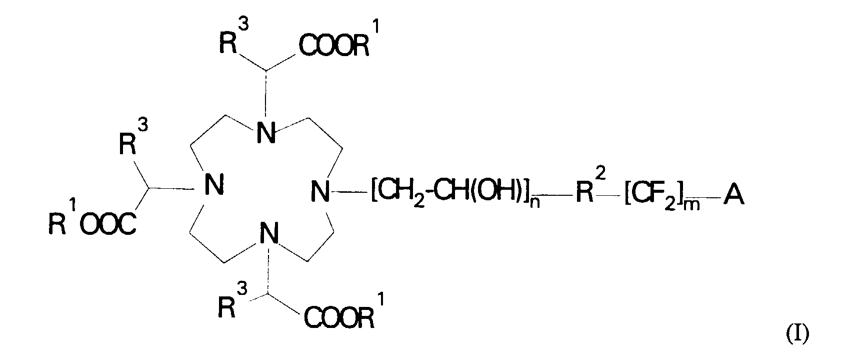 worin
n und m unabhängig voneinander für die Ziffern 0 oder 1 steht,
R¹ unabhängig voneinander für ein Wasserstoffatom oder ein Metallionenäquivalent,
R³ für ein Wasserstoffatom, eine geradkettige oder verzweigte C₁-C₁₀ Alkylgruppe, die gegebenenfalls durch 1-5 C₁-C₆-Alkoxygruppen, Hydroxy-C₁-C₆-alkylgruppen und/oder Hydroxygruppen substituiert ist,
R² für eine durch 1 bis 3 -CF₃ - Gruppen substituierte geradkettige oder verzweigte C₁-C₁₀ Alkylengruppe, die gegebenenfalls durch 1 bis 5 Sauerstoffatome und oder Carbonylgruppen unterbrochen ist und/oder gegebenenfalls durch 1 bis 5 Hydroxygruppen, C₁-C₆-Alkoxy-C₁-C₆-alkylgruppen, -OR⁴ -, -CO-NR⁵R⁶-, -NR⁵R⁶ - und/oder -NR⁵-CO-R⁶ - Reste substituiert ist,
worin R⁴ für einen geradkettigen oder verzweigten C₁-C₄ Alkylrest steht und R⁵, R⁶ unabhängig voneinander die Bedeutung von R³ haben und
A für den Fall, daß m die Ziffer 1 bedeutet, für ein Fluoratom steht, im Falle daß m die Ziffer 0 bedeutet, für ein Wasserstoffatom oder einen zweiten makrocyclischen Rest der allgemeinen Formel II steht, 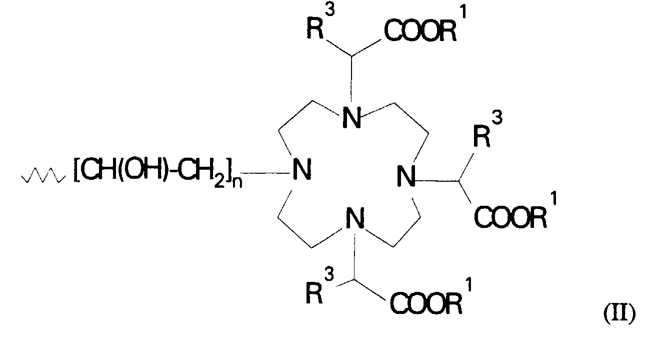
worin
n, R¹ und R³ die angegebenen Bedeutungen haben,
sowie deren Salzen mit anorganischen und/oder organischen Basen oder Aminosäuren und/oder deren C₁-C₆-Alkyl- oder C₆-C₁₀-Aryl- oder Aralky-Estern oder Amiden, wobei mindestens zwei Reste R¹ für ein Metallionenäquivalent stehen.

2. Fluorhaltige makrocyclischen Metallkomplexen gemäß Anspruch 1 enthaltend als Rest 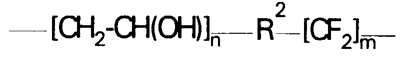 eine -CH₂-CH(OH)-CF₃-, -CH₂-C(OH)CF₃-CH₃ -, -CH₂-CH(OH)-C(CF₃)₃ -oder -CH₂-CH(OH)-CH₂O-C(CF₃)₃-Gruppe.

3. Verwendung von fluorhaltigen, makrocyclischen Metallkomplexen gemäß Anspruch 1 in der NMR-Spektroskopie.

4. Verwendung von fluorhaltigen, makrocyclischen Metallkomplexen gemäß Anspruch 1 als Temperatursonden zur Bestimmung der Gewebetemperatur mittels NMR-Spektroskopie.

5. Diagnostische Mittel zur Bestimmung der Gewebetemperatur, enthaltend mindestens eine Komplexverbindung nach Anspruch 1.

6. Verfahren zur Herstellung von fluorhaltigen makrocyclischen Metallkomplexen bestehend aus mindestens einem Metallion eines Elementes der Ordnungszahlen 21-29, 42, 44 oder 57-83 und einem Komplexbildner der allgemeinen Formel I 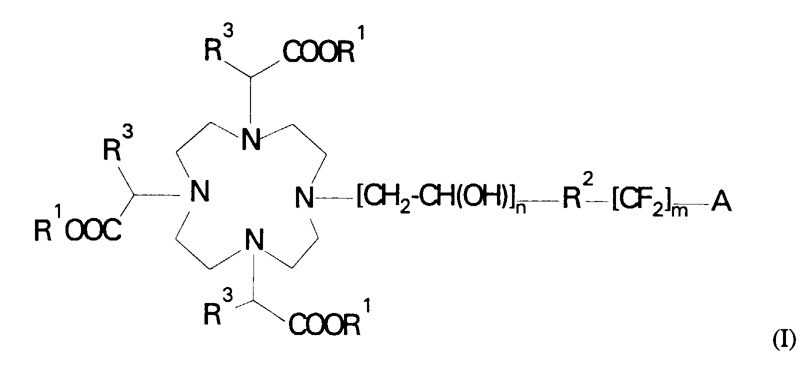 worin
n und m unabhängig voneinander für die Ziffern 0 oder 1 steht,
R¹ unabhängig voneinander für ein Wasserstoffatom oder ein Metallionenäquivalent,
R³ für ein Wasserstoffatom, eine geradkettige oder verzweigte C₁-C₁₀ Alkylgruppe, die gegebenenfalls durch 1-5 C₁-C₆-Alkoxygruppen, Hydroxy-C₁-C₆-alkylgruppen und/oder Hydroxygruppen substituiert ist,
R² für eine durch 1 bis 3 -CF₃ - Gruppen substituierte geradkettige oder verzweigte C₁-C₁₀ Alkylengruppe, die gegebenenfalls durch 1 bis 5 Sauerstoffatome und oder Carbonylgruppen unterbrochen ist und/oder gegebenenfalls durch 1 bis 5 Hydroxygruppen, C₁-C₆-Alkoxy-C₁-C₆-alkylgruppen, -OR⁴ -, -CO-NR⁵R⁶ -, -NR⁵R⁶ - und/oder -NR⁵-CO-R⁶ - Reste substituiert ist,
worin R⁴ für einen geradkettigen oder verzweigten C₁-C₄ Alkylrest steht und R⁵, R⁶ unabhängig voneinander die Bedeutung von R³ haben und
A für den Fall, daß m die Ziffer 1 bedeutet, für ein Fluoratom steht, im Falle daß m die Ziffer 0 bedeutet, für ein Wasserstoffatom oder einen zweiten makrocyclischen Rest der allgemeinen Formel II steht, 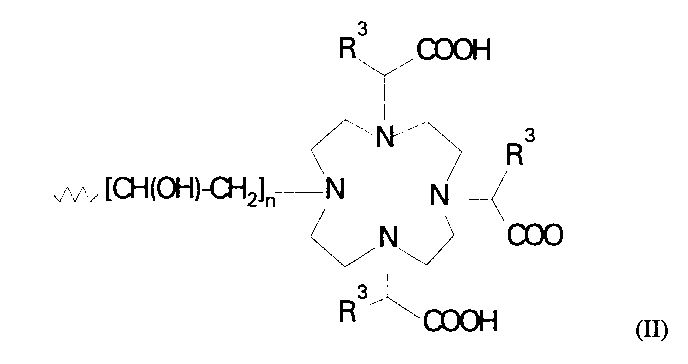
worin
n, R¹ und R³ die angegebenen Bedeutungen haben,
sowie deren Salze mit anorganischen und/oder organischen Basen oder Aminosäuren, und/oder deren C₁-C₆-Alkyl- oder C₆-C₁₀-Aryl- oder Aralky-Estern oder Amiden, wobei mindestens zwei Reste R¹ für ein Metallionenäquivalent stehen,
dadurch gekennzeichnet, daß man die Komplexbildner der allgemeinen Formel IV 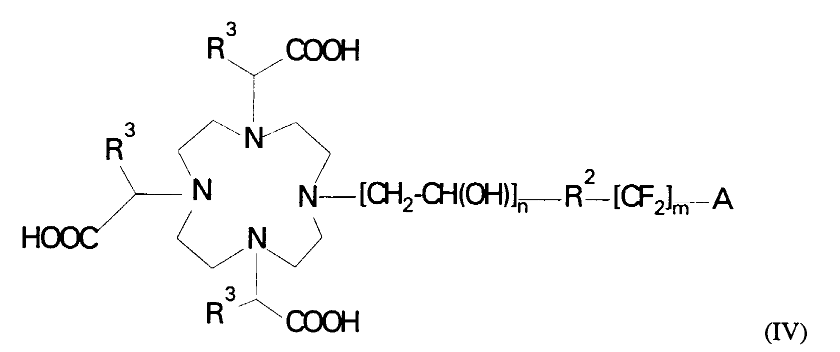 worin n, m, R² und R³ die angegebene Bedeutung haben und A' für den Fall, daß m die Ziffer 1 bedeutet für ein Fluoratom steht und im Falle, daß m die Ziffer 0 bedeutet, für ein Wasserstoffatom oder einen zweiten makrocyclischen Rest der Formel V steht 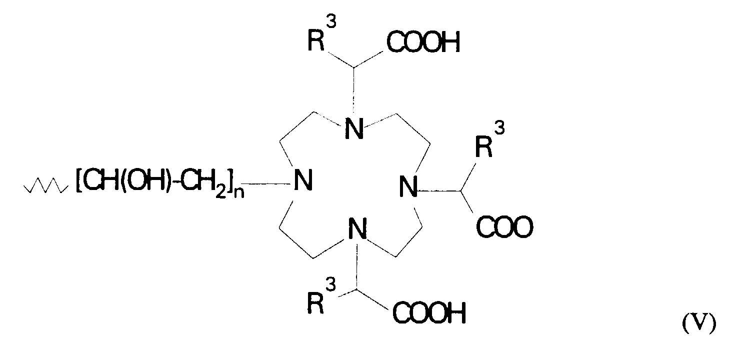 in bekannter Weise mit einem Metallsalz oder Metalloxid eines Elementes der Ordnungszahlen 21-29, 42, 44 oder 57-83 zu den gewünschten Metallkomplexen umsetzt.

7. Verfahren zur Herstellung von pharmazeutischen Mitteln gemäß Anspruch 6, dadurch gekennzeichnet, daß das in Wasser gelöste Komplexsalz mit den in der Galenik üblichen Zusätzen bzw. Stabilisatoren in eine für die enterale bzw. parenterale Applikation geeignete Form gebracht wird, so daß das Komplexsalz in einer Konzentration von 1µm bis 1 mol/l vorliegt.

## Claims

1. Fluorine-containing macrocyclic metal complexes consisting of at least one metal ion of an element of atomic number 21-29, 42, 44 or 57-83 and a complexing agent of the general formula I 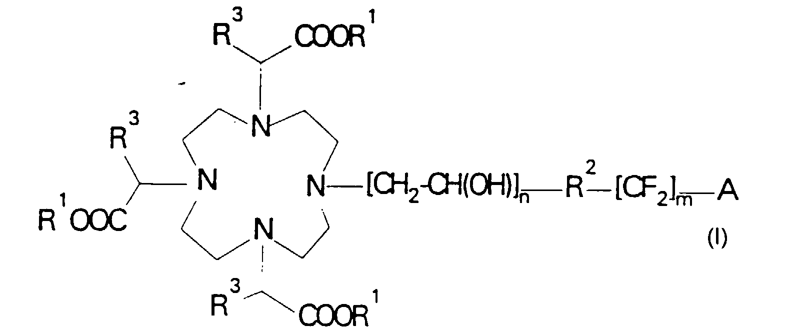 in which
n and m, each independently of the other, represent the number 0 or 1,
the radicals R¹, each independently of the others, represent a hydrogen atom or a metal ion equivalent,
the radicals R³ represent a hydrogen atom, or a straight-chained or branched C₁-C₁₀alkyl group that is optionally substituted by from 1 to 5 C₁-C₆alkoxy groups, hydroxy-C₁-C₆alkyl groups and/or hydroxy groups,
R² represents a straight-chained or branched C₁-C₁₀alkylene group substituted by from 1 to 3 -CF₃- groups, which C₁-C₁₀alkylene group is optionally interrupted by from 1 to 5 oxygen atoms and/or carbonyl groups and/or is optionally substituted by from to 5 hydroxy groups, C₁-C₆alkoxy-C₁-C₆alkyl groups, -OR⁴- radicals, -CO-NR⁵R⁶- radicals, -NR⁵R⁶- radicals and/or -NR⁵-CO-R⁶- radicals,
wherein R⁴ represents a straight-chained or branched C₁-C₄alkyl radical and R⁵ and R⁶, each independently of the other, have the meaning of R³, and
A is a fluorine atom when m is the number 1 and, when m is the number 0, A is a hydrogen atom or a second macrocyclic radical of the general formula II 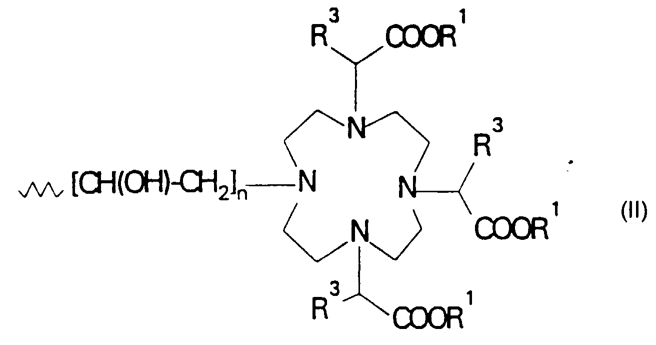 in which
n, R¹ and R³ have the meanings given,
and their salts with inorganic and/or organic bases or amino acids, and/or their C₁-C₆alkyl or C₆-C₁₀aryl or aralkyl esters or amides, wherein at least two radicals R¹ are metal ion equivalents.

2. Fluorine-containing macrocyclic metal complexes according to claim 1 containing as the radical 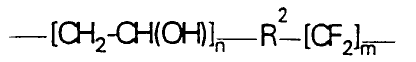 a group CH₂-CH(OH)-CF₃-, -CH₂-C(OH)CF₃-CH₃-, -CH₂-CH(OH)-C(CF₃)₃- or -CH₂-CH(OH)-CH₂O-C(CF₃)₃-.

3. Use of fluorine-containing macrocyclic metal complexes according to claim 1 in NMR spectroscopy.

4. Use of fluorine-containing macrocyclic metal complexes according to claim 1 as temperature probes for determining the temperature of tissue by means of NMR spectroscopy.

5. Diagnostic compositions for determining the temperature of tissue, containing at least one complex compound according to claim 1.

6. Process for the preparation of fluorine-containing macrocyclic metal complexes consisting of at least one metal ion of an element of atomic number 21-29, 42, 44 or 57-83 and a complexing agent of the general formula I 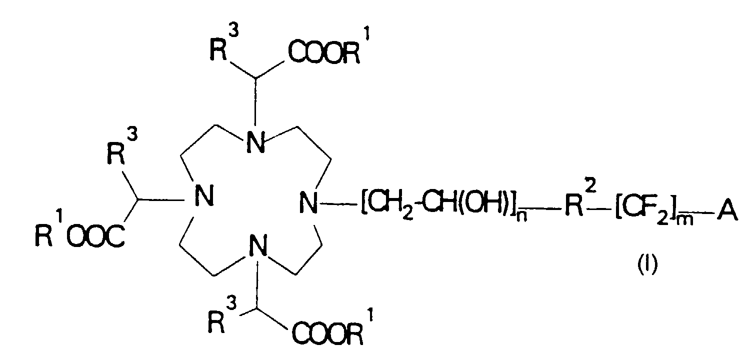 in which
n and m, each independently of the other, represent the number 0 or 1,
the radicals R¹, each independently of the others, represent a hydrogen atom or a metal ion equivalent,
the radicals R³ represent a hydrogen atom, or a straight-chained or branched C₁-C₁₀alkyl group that is optionally substituted by from 1 to 5 C₁-C₆alkoxy groups, hydroxy-C₁-C₆alkyl groups and/or hydroxy groups,
R² represents a straight-chained or branched C₁-C₁₀alkylene group substituted by from 1 to 3 -CF₃- groups, which C₁-C₁₀alkylene group is optionally interrupted by from 1 to 5 oxygen atoms and/or carbonyl groups and/or is optionally substituted by from 1 to 5 hydroxy groups, C₁-C₆alkoxy-C₁-C₆alkyl groups, -OR⁴- radicals, -CO-NR⁵R⁶- radicals, -NR⁵R⁶- radicals and/or -NR⁵-CO-R⁶- radicals,
wherein R⁴ represents a straight-chained or branched C₁-C₄alkyl radical and R⁵ and R⁶, each independently of the other, have the meaning of R³, and
A is a fluorine atom when m is the number 1 and, when m is the number 0, A is a hydrogen atom or a second macrocyclic radical of the general formula II 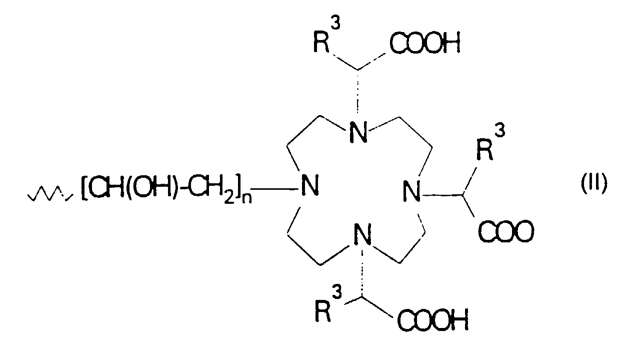 in which
n, R¹ and R³ have the meanings given,
and their salts with inorganic and/or organic bases or amino acids, and/or their C₁-C₆alkyl or C₆-C₁₀aryl or aralkyl esters or amides, wherein at least two radicals R¹ are metal ion equivalents,
characterised in that the complexing agents of the general formula IV 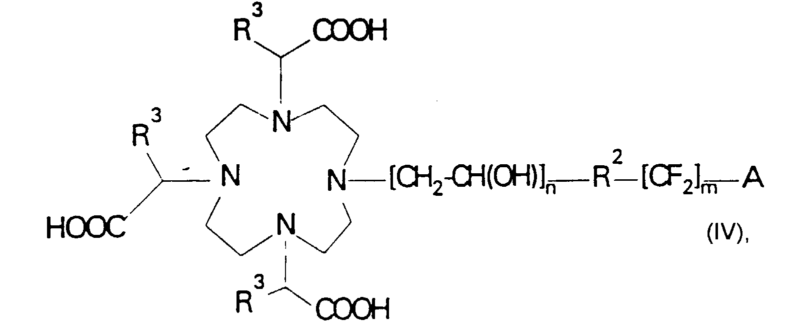 in which n, m, R² and R³ have the meanings given, and A' is a fluorine atom when m is the number 1 and, when m is the number 0, A' is a hydrogen atom or a second macrocyclic radical of formula V 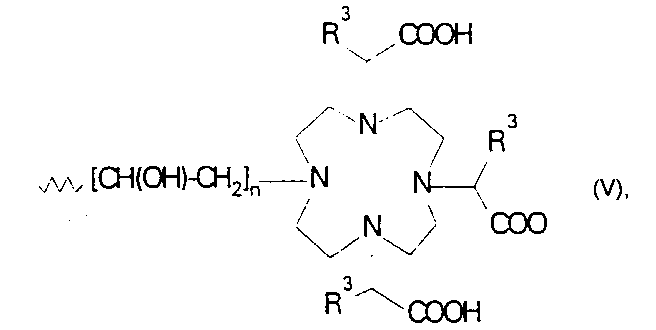 are reacted in known manner with a metal salt or metal oxide of an element of atomic number 21-29, 42, 44 or 57-83 to form the desired metal complexes.

7. Process for the preparation of pharmaceutical compositions according to claim 6, characterised in that the complex salt, dissolved in water, together with additives and stabilisers customary in galenical pharmacy, is brought into a form suitable for enteral or parenteral administration, in such a manner that the complex salt is present in a concentration of from 1 µm to 1 mol/litre.

## Revendications

1. Complexes métalliques macrocycliques fluorés composés d'au moins un ion métallique d'un élément avec un numéro atomique 21-29, 42, 44 ou 57-83 et d'un agent complexant de formule générale I 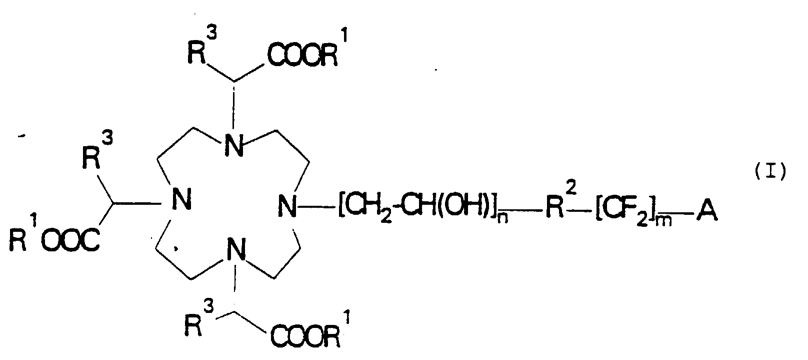 dans laquelle
n et m indépendamment l'un de l'autre, valent 0 ou 1,
R¹ indépendamment l'un de l'autre, représentent un atome d'hydrogène ou un équivalent d'ion métallique,
R³ représente un atome d'hydrogène, un groupe alkyle en C₁-C₁₀, linéaire ou ramifié, qui est éventuellement substitué par 1 à 5 groupes alcoxy en C₁-C₆, groupes hydroxyalkyle en C₁-C₆ et/ou groupes hydroxy,
R² représente un groupe alkylène en C₁-C₁₀, linéaire ou ramifié, éventuellement substitué par 1 à 3 groupes CF₃, lequel groupe alkylène est éventuellement interrompu par 1 à 5 atomes d'oxygène et/ou des groupes carbonyle et/ou éventuellement substitué par 1 à 5 groupes hydroxy, groupes (alcoxy en C₁-C₆)-(alkyle en C₁-C₆), les restes -OR⁴-, -CO-NR⁵R⁶-, -NR⁵R⁶- et/ou -NR⁵-CO-R⁶,
où R⁴ est un reste alkyle en C₁-C₄, linéaire ou ramifié,, et R⁵, R⁶, indépendamment l'un de l'autre, ont la signification de R³, et
A dans le cas où m vaut 1, représente un atome de fluor, dans le cas où m vaut 0, un atome d'hydrogène ou un deuxième reste macrocyclique de formule générale II, 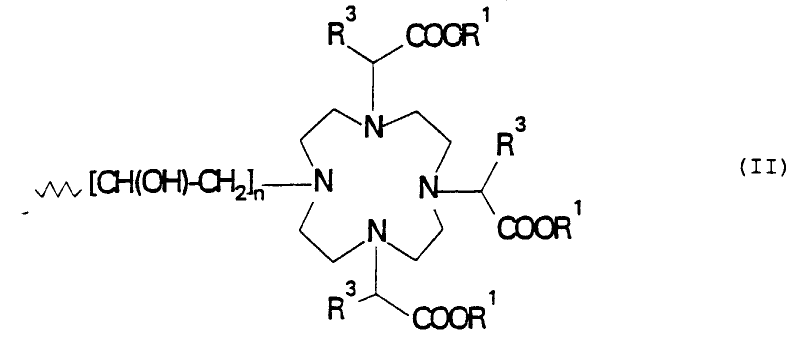
dans laquelle
n, R¹ et R³ ont les significations données,
ainsi que leurs sels avec des bases inorganiques et/ou organiques et des acides aminés et/ou leurs amides ou esters d'alkyles en C₁-C₆ ou d'aralkyles ou d'aryles en C₆-C₁₀, au moins deux restes R¹ étant un équivalent d'ion métallique.

2. Complexes métalliques macrocycliques fluorés selon la revendication 1, comprenant en tant que reste
-[CH₂-CH(OH)]ₙ-R-[CF₂]ₘ- A
représente un groupe -CH₂-CH(OH)-CF₃, -CH₂-C(OH)CF₃-CH₃, -CH₂-CH(OH)-C(CF₃)₃ ou -CH₂-CH(OH) -CH₂O-C(CF₃)₃.

3. Utilisation de complexes métalliques macrocycliques fluorés selon la revendication 1 dans la spectroscopie par la RMN.

4. Utilisation de complexes métalliques macrocycliques fluorés selon la revendication 1, en tant que sondes de température pour la détermination de la température tissulaire à l'aide de la spectroscopie par la RMN.

5. Produits diagnostiques pour déterminer la température tissulaire contenant au moins un composé complexe selon la revendication 1.

6. Procédé pour préparer des complexes métalliques macrocycliques fluorés, composés d'au moins un ion métallique d'un élément avec le numéro atomique 21-29, 42, 44 ou 57-83 et d'un agent complexant de formule générale I 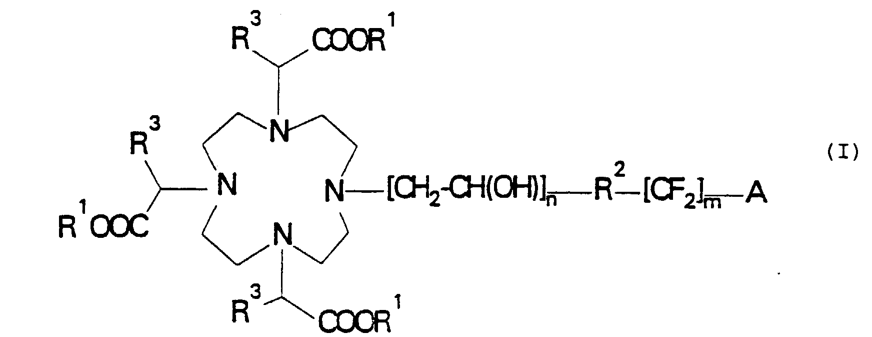 dans laquelle
n et m, indépendamment l'un de l'autre, valent 0 ou 1,
R¹ indépendamment l'un de l'autre, représentent un atome d'hydrogène ou un équivalent d'ion métallique,
R³ représente un atome d'hydrogène, un groupe alkyle en C₁-C₁₀, linéaire ou ramifié, qui est éventuellement substitué par 1 à 5 groupes alcoxy en C₁-C₆, groupes hydroxyalkyle en C₁-C₆ et/ou groupes hydroxy,
R² représente un groupe alkylène en C₁-C₁₀, linéaire ou ramifié, éventuellement substitué par 1 à 3 groupes CF₃, lequel groupe alkylène est éventuellement interrompu par 1 à 5 atomes d'oxygène et/ou des groupes carbonyle et/ou éventuellement substitué par 1 à 5 groupes hydroxy, groupes (alcoxy en C₁-C₆)-(alkyle en C₁-C₆), les restes -OR⁴-, -CO-NR⁵R⁶-, -NR⁵R⁶- et/ou -NR⁵-CO-R⁶,
où R⁴ est un reste alkyle en C₁-C₄ linéaire ou ramifié et R⁵, R⁶, indépendamment l'un de l'autre, ont la signification de R³, et
A dans le cas où m vaut 1, représente un atome de fluor, dans le cas où m vaut 0, un atome d'hydrogène ou un deuxième reste macrocyclique de formule générale II, 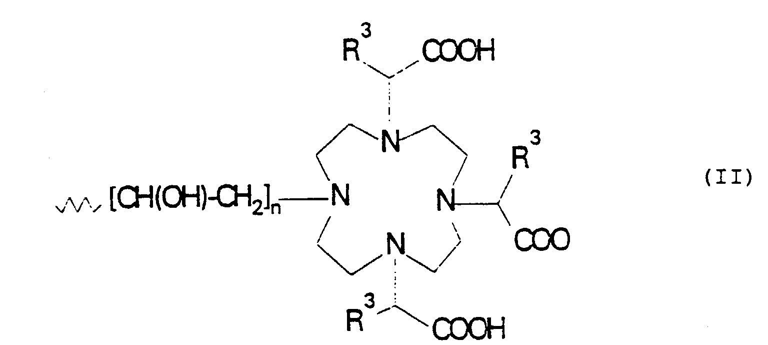
dans laquelle
n, R¹ et R³ ont les significations données,
ainsi que leurs sels avec des bases inorganiques et/ou organiques ou des acides aminés et/ou leurs amides ou esters d'alkyles en C₁-C₆ ou d'aralkyles ou d'aryles en C₆-C₁₀, au moins deux restes R¹ étant un équivalent d'ion métallique, caractérisé en ce que l'on fait réagir l'agent complexant de formule générale IV 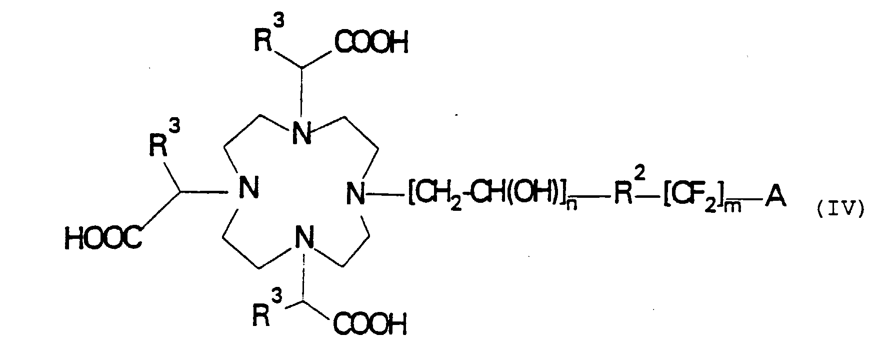 dans laquelle n, m, R² et R³ ont les significations données et A', dans le cas où m vaut 1, représente un atome de fluor et dans le cas où m vaut 0, représente un atome d'hydrogène ou un deuxième reste, macrocyclique de formule V, 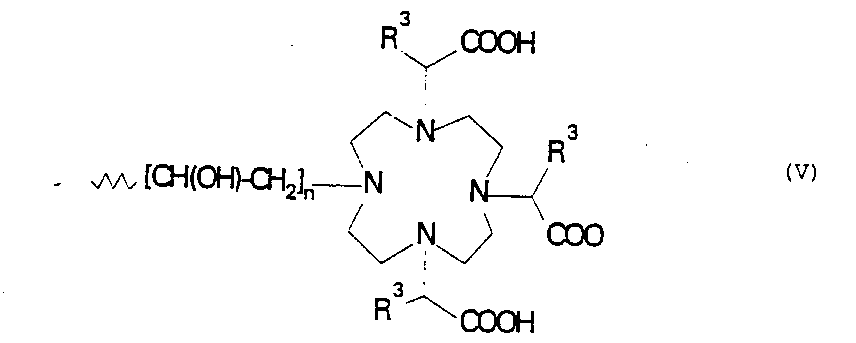 de manière connue en soi, avec un sel métallique ou un oxyde métallique d'un élément avec un numéro atomique 21-29, 42, 44 ou 57-83 pour obtenir des complexes métalliques voulus.

7. Procédé pour la préparation de produits pharmaceutiques selon la revendication 6, caractérisé en ce que l'on met en forme appropriée aux applications entérales ou bien parentérales le sel complexe dissous dans l'eau avec des adjuvants ou stabilisants usuels en galénique, de façon telle que le sel complexe soit présent à raison de 1 µmole à 1 mole/litre.
